# EUROPÄISCHE PATENTSCHRIFT

(11) **EP 2 176 141 B1**
(45) Veröffentlichungstag und Bekanntmachung des Hinweises auf die Patenterteilung: **02.01.2013**
(21) Anmeldenummer: 08757962.9
(22) Anmeldetag: 19.04.2008
(51) Int. Cl.: A47G 19/22, A61M 31/00, B65D 83/16, B65D 83/28

(54) **APPLIKATORSYSTEM MIT EINEM AEROSOLBEHÄLTER**
APPLICATOR SYSTEM HAVING AN AEROSOL TANK
SYSTÈME APPLICATEUR COMPRENANT UN RÉSERVOIR AÉROSOL

(30) Priorität: 18.05.2007 DE 102007023235
(43) Veröffentlichungstag der Anmeldung: 21.04.2010
(73) Patentinhaber: ColepCCL Laupheim GmbH & Co. KG, 88471 Laupheim (DE)
(72) Erfinder: WITTE, Alexander, 26384 Wilhelmshaven (DE)
(74) Vertreter: Hauck Patent- und Rechtsanwälte
(86) Internationale Anmeldenummer: PCT/DE2008/000661
(87) Internationale Veröffentlichungsnummer: WO 2008/141600

(56) Entgegenhaltungen:
- WO-A-00/16837
- US-A1- 2004 020 486

## Beschreibung

Die vorliegende Erfindung betrifft ein Applikatorsystem mit einem Aerosolbehälter, der ein unter Druck stehendes Medium und ein Ventil zum Austragen des Mediums - aufweist.

Im folgenden wird mit Aerosolbehälter stets auf eine Flasche, Dose oder einen sonstigen Behälter abgestellt, die bzw. der über ein Ventil verschlossen ist. Wird das Ventil betätigt, tritt das Medium aus der Flasche bzw. dem sonstigen Behälter über das Ventil aus.

Bei dem Ventil kann es sich um ein herkömmliches Aerosolventil handeln, beispielsweise Kugel- oder Beutelventile und Dosierventile. Auch können herkömmliche Aerosolflaschen oder Aerosoldosen beispielsweise aus Weißblech oder Aluminium eingesetzt werden. US 2004/020486 zeigt ein gatlungs-gemäßes Ventil.

Aus US 2006/0226110 A1 ist eine Trinkflasche mit einem bißaktivierten Mundstück bekannt. Bei der Trinkflasche handelt es sich nicht um einen unter Druck stehenden Aerosolbehälter, sondern um eine herkömmliche Trinkflasche, in die ein Trinkhalm eintaucht. Auf dem Ende des Trinkhalms ist ein bißaktiviertes Mundstück angeordnet, das sich als Kippventil unter der Kraft des Bisses elastisch öffnet. Ohne Krafteinwirkung auf das Mundstück kehrt dieses elastisch in seine Verschlußposition zurück.

Aus US 2002/0038807 A1 ist ein Mundstück zum Trinken bekannt. Das Mundstück besitzt einen Austrittskanal für die Flüssigkeit, wobei ein Ventil in das Mundstück integriert ist und aus seiner Verschlußstellung gegen eine Federkraft zurückschiebbar ist. Zur Benutzung ist ein Behälter vorgesehen, aus dem über einen Schlauch dem Mundstück Flüssigkeit zugeführt wird. Zum Öffnen wird das Mundstück von einem Benutzer in den Mund genommen und mit den Zähnen aus seiner verschlossenen Position in die geöffnete Position geschoben. Anschließend kann über das geöffnete Mundstück Flüssigkeit aus dem Behälter gesaugt werden.

Aus US 6,010,034 ist eine Kombination eines Sprühbehälters und einer zusätzlichen Trinkflasche bekannt. Der Sprühbehälter versprüht über ein handbetätigtes Pumpsystem die Flüssigkeit.

Aus US 5,062,591 ist ein Mundstück für ein Trinksystem bekannt. Das Mundstück besitzt ein Kugelventil, dessen Kugel über eine Feder in den Ventilsitz gedrückt ist. Zum Öffnen des Ventils muß der Benutzer auf das Mundstück beißen, um dieses zu verformen. Auf diese Weise entsteht ein Flüssigkeitsdurchgang, der die Entnahme von Flüssigkeit erlaubt.

Aus US 4,457,711 ist Mundhygienespray bekannt, das einen unter Druck stehenden Behälter mit einem Ventil aufweist. Der Sprühkopf besitzt ein elastisches konkaves Mundstück, das auf den Sprühkopf aufgesetzt ist. Zur Betätigung weist der Sprühkopf ein Betätigungsfeld auf, das von Hand betätigt das Ventil öffnet, um die Zähne eines Benutzers mechanisch durch einen Strahl des austretenden Fluids zu reinigen.

Aus EP 0 266 067 A1 ist ein bißbetätigtes Trinkventil bekannt, das an einen Trinkschlauch angeschlossen wird und diesen verschließt. Durch einen Biß auf das Mundstück wird das Ventil geöffnet, so daß Flüssigkeit über den Trinkschlauch angesaugt werden kann.

Der Erfindung liegt die Aufgabe zugrunde, ein Applikatorsystem mit einem Aerosolbehälter zum Austrag eines Mediums bereitzustellen, das zur oralen Flüssigkeits - und Vital-/Nährstoffversorgung sowie zur oralen Zuführung von medizinischen Wirkstoffen eingesetzt werden kann und zugleich eine einfache Betätigung durch den Benutzer erlaubt.

Erfindungsgemäß wird die Aufgabe durch ein Applikatorsystem mit den Merkmalen nach Anspruch 1 gelöst. Vorteilhafte Ausgestaltungen bilden den Gegenstand der Unteransprüche.

Das erfindungsgemäße Applikatorsystem besitzt einen Aerosolbehälter mit einem unter Druck stehenden Medium und weist ein Ventil auf, über das das Medium bei Betätigung des Ventils ausgetragen wird. Der Sprühkopf weist einen Sprühkopfkörper auf, der einen mit dem Ventil verbundenen Austrittskanal besitzt und eine Austrittsöffnung, in der der Austrittskanal mündet. Ferner ist ein Mundelement erfindungsgemäß vorgesehen, das auf dem Sprühkopfkörper sitzt. Die Austrittsöffnung und das Mundelement sind relativ zueinander angeordnet, um die Austrittsöffnung an den Mund zu führen und gleichzeitig über das Mundelement das Ventil betätigen zu können. Im Gegensatz zu bekannten Mundstücken wird erfindungsgemäß das Ventil des Aerosolbehälters so betätigt, daß das Medium über die Austrittsöffnung in den Mund des Benutzers gesprüht wird.

Mit dem erfindungsgemäß ausgestalteten Applikatorsystem erfolgt die Abgabe des Mediums direkt in den Mund des Benutzers, wobei gleichzeitig der Benutzer über den Mund und den auf das Mundelement ausgeübten Druck das Ventil zur Abgabe des Mediums öffnet. Das erfindungsgemäße Applikatorsystem gestattet damit eine effektive orale Aufnahme von Flüssigkeit bei einer sehr einfachen Handhabung, so daß das System insbesondere auch für Sportler geeignet ist. Hinzu kommt, daß eine Gefahr des Verschluckens bei der Flüssigkeitsaufhahme minimiert wird, da die Flüssigkeit nicht aus einem Flüssigkeitsbehälter gesaugt werden muß. Auch kann der Benutzer des erfindungsgemäßen Applikatorsystems zudem die auszutragende Flüssigkeitsmenge kontrollieren.

In einer vorteilhaften Ausgestaltung des erfindungsgemäßen Applikatorsystems weist das Mundelement eine zentrale Öffnung auf, in die die Austrittsöffnung mündet. Bevorzugt ist die Austrittsöffnung in einem Abgabevorsprung angeordnet, der durch die zentrale Öffnung in dem Mundelement vorsteht. Bei der Benutzung kann der vorstehende Abgabevorsprung von dem Benutzer mit den Lippen umschlossen werden, so daß die Flüssigkeit direkt in den Mund eintritt.

Für das Mundelement sind grundsätzlich unterschiedliche Ausgestaltungen denkbar. Wichtig an dem Mundelement ist, daß über das Mundelement ein gewählter Druck auf den Sprühkopfkörper ausgeübt werden kann, um so das Ventil des Aerosolbehälters zu öffnen.

Unterstützend zu der bereits angesprochenen Betätigung über den Mund kann das Applikatorsystem auch ergänzend oder ausschließlich von Hand betätigt werden. Hierzu sind bevorzugt Griffbereiche an dem Mundelement und/oder dem Sprühkopf vorgesehen, die bei einer manuellen Betätigung ergriffen werden. Zweckmäßigerweise sind die Griffbereiche als vorstehende Ringe ausgebildet.

In einer bevorzugten Ausgestaltung besitzt das Mundelement einen gewölbten Schild. Der Schild ist bevorzugt mit zwei flachen von der Austrittsöffnung fortweisenden Flügeln ausgestattet, wobei bevorzugt zwei Flügel vorgesehen sind. Der Schild liegt bei der Benutzung ganz oder teilweise an den Lippen des Benutzcrs an.

In einer alternativen Ausgestaltung kann das Mundelement eine oder mehrere Angriffsflächen zum Beißen aufweisen. Die Angriffsflächen oder Bißflächen werden zur Kraftübertragung auf das Ventil des Aerosolbehälters beißend erfaßt und so die notwendige Kraft auf das Ventil ausgeübt.

Unabhängig von der konkreten Ausgestaltung des Mundelements wird eine über das Mundelement in axialer Richtung des Aerosolbehälters aufgebrachte Kraft auf das Ventil weitergeleitet. Das Ventil ist entsprechend ausgelegt, um bei einem leichten Druck über den Mund betätigt zu werden und zu öffnen und bei nachlassendem Druck selbsttätig zu schließen.

In einer bevorzugten Ausgestaltung besitzt der Sprühkopfkörper eine umlaufende Wand, die einen Befestigungsabschnitt für das Mundelement bildet. Bevorzugt weist das Mundelement einen zylindrischen Ansatzbereich auf, der ebenfalls eine umlaufende Wand besitzt. Die Innenseite der umlaufenden Wand des Ansatzbereichs liegt im aufgesetzten Zustand an der Außenseite des Befestigungsabschnitts an dem Sprühkopfkörper an. Bevorzugt besitzt der Befestigungsabschnitt einen umlaufenden Absatz, an dem der Ansatzbereich des Mundelements aufliegt.

Mundelement und Sprühkopf bzw. Sprühkopfkörper sowie weitere Elemente können mehrteilig oder alternativ auch einteilig ausgebildet sein.

In einer bevorzugten Ausgestaltung wird die ausgetragene Flüssigkeit zerstäubt. Zweckmäßigerweise ist hierzu ein Sprühelement in dem Austrittskanal des Sprühkopfs angeordnet. Das Sprühelement ist vorgesehen, um das Austragsverhalten des Mediums zu verändern, insbesondere um das auszutragende Medium zu versprühen. Das Sprühelement kann bevorzugt als Einsetzkörper ausgebildet sein, der in dem Abgabevorsprung oder kurz vor der Austrittsöffnung angeordnet ist.

Bevorzugte Ausführungsbeispiele des erfindungsgemäßen Aerosolbehälters werden nachfolgend anhand von Zeichnungen näher erläutert.
- Fign. 1 a und b: zeigen einen Aerosolbehälter mit einem ersten Mundelement in einer perspektivischen Ansicht, wobei Fig. 1a ein Kugelventil mit Steigleitung und Fig. 1b ein Beutelventil besitzt,
- Fign. 2 a und b: zeigen einen weiteren Aerosolbehälter mit einem zweiten Mundelement, wobei Fig. 2a ein Kugelventil mit Steigleitung und Fig. 2b ein Beutelventil aufweist,
- Fign. 3 bis 5: zeigen perspektivische Ansichten des Sprühkopfs mit dem ersten Mundelement, und
- Fign. 6 bis 8: zeigen Ansichten des Sprühkopfs mit dem zweiten Mundelement.

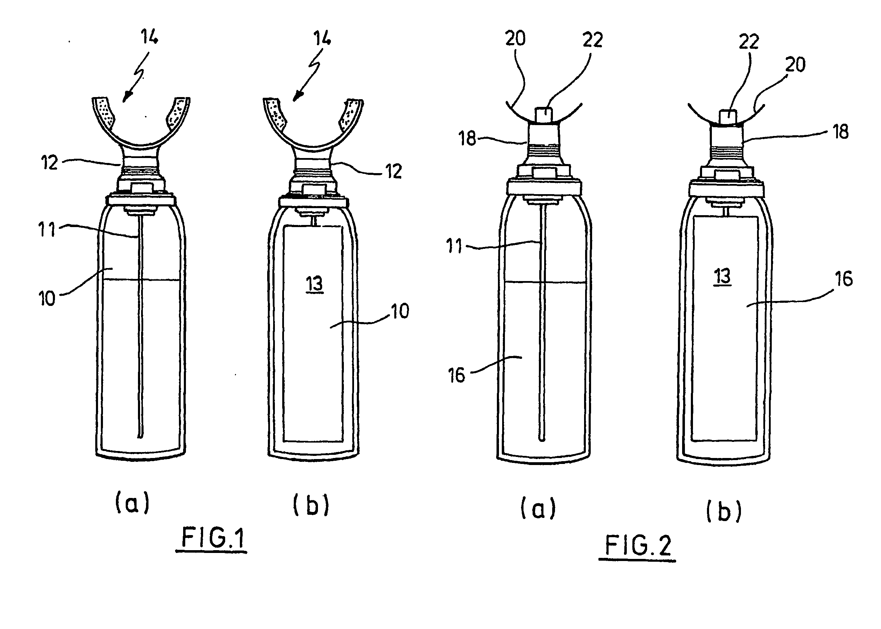

Fign. 1 a und b zeigen jeweils ein Applikatorsystem mit einer unter Druck stehenden Flasche 10, auf der ein Sprühkopf 12 mit einem ersten Mundelement 14 angeordnet ist. Bei der Flasche 10 kann es sich um eine Metallflasche beispielsweise aus Weißblech oder Aluminium handeln. In der Ausgestaltung aus Fig. 1a ist ein Kugelventil mit einer Steigleitung 11 vorgesehen. In Fig. 1b ist das auszutragende Medium in einem Beutel 13 vorgesehen, der zu einem sogenannten Beutelventil bag-on-valve gehört.

Fign. 2a und b zeigen jeweils ein erfindungsgemäßes Applikatorsystem in einer zweiten Ausgestaltung. Auch hier ist eine Aerosolflasche 16 aus Metall vorgesehen, auf die ein Sprühkopf 18 mit einem zweiten Mundelement 20 aufgesetzt ist. Im zentralen Bereich des Mundelements 20 ist ein Abgabevorsprung 22 zu erkennen.

Neben den dargestellten Kugel- und Beuteventilen können bei der Erfindung auch Dossierventile oder Dosierköpfe eingesetzt werden, um die Menge an abgegebener Flüssigkeit zu dosieren.

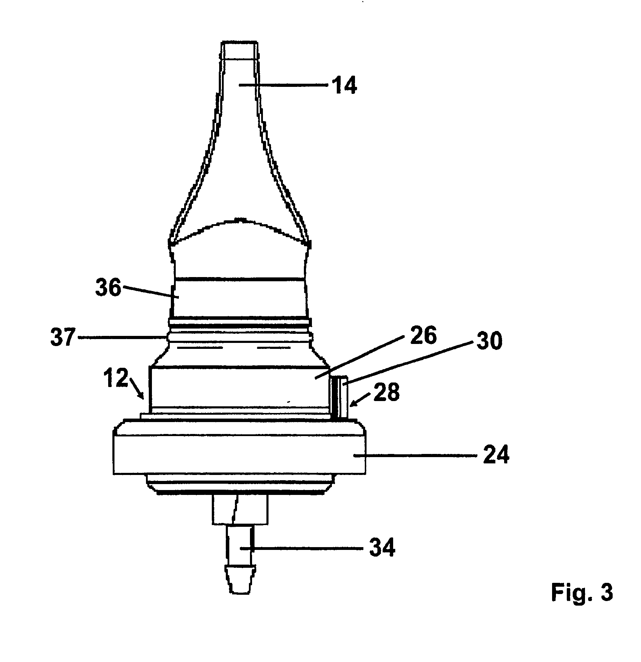

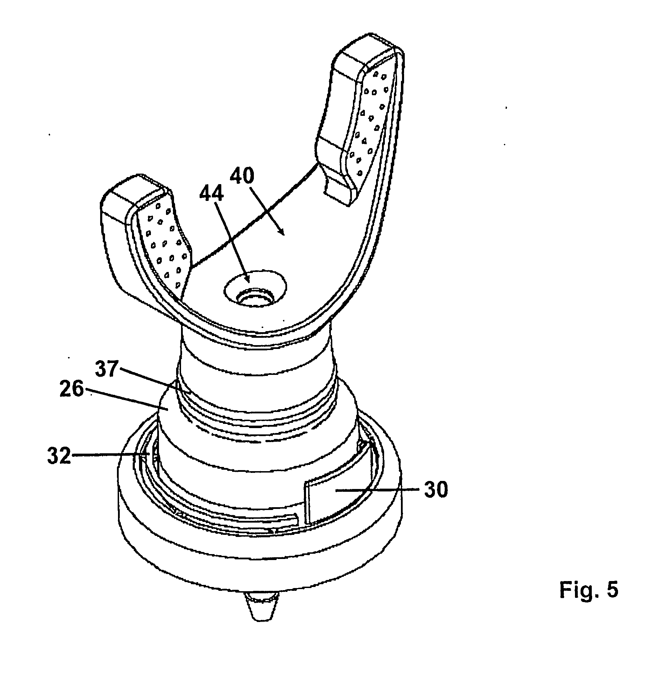

Fign. 3 bis 5 zeigen den Sprühkopf mit dem ersten Mundelement 14 aus Fig. 1. Der Sprühkopf 12 besitzt einen Sprühkopfkörper 26 und einen umlaufenden Befestigungsring 24, der auf die Aerosolflasche aufgesetzt ist. Der Befestigungsring 24 kann beispielsweise auf den Rand eines Ventiltellers aufgesetzt sein. Zwischen Befestigungsring 24 und Sprühkopfkörper 26 befindet sich als zusätzliches Sicherungselement ein Originalitätsverschluß 28. Der Originalitätsverschluß 28 besitzt eine Lasche 30 und ein umlaufendes Band 32, das über Stege mit dem Befestigungsring 24 und den Sprühkopfkörper 26 verbunden ist. In seinem verschlossenen Zustand sichern die Stege des Originalitätsverschlusses den Sprühkopfkörper 26 an dem Befestigungsring 24 und verhindern so eine Betätigung des Sprühkopfs. Der Sprühkopfkörper 26 ist in dem dargestellten Ausführungsbeispiel mit einem Stem 34 ausgestattet, der für eine Verbindung mit einem weiblichen Ventil vorgesehen ist. Grundsätzlich kann der Sprühkopf zur Verbindung mit jeglichem Ventil ausgelegt sein.

Auf den Sprühkopf aufgesetzt ist ein Mundelement 14. Das Mundelement 14 besitzt einen Ansatzbereich 36, der als zylindrischer Ansatzstutzen ausgebildet ist. Der Ansatzstutzen ist auf einem oberen Befestigungsabschnitt des Sprühkopfkörpers 26 aufgesetzt. Alternativ können Sprühkopfkörper und Mundteil auch einteilig ausgestaltet sein.

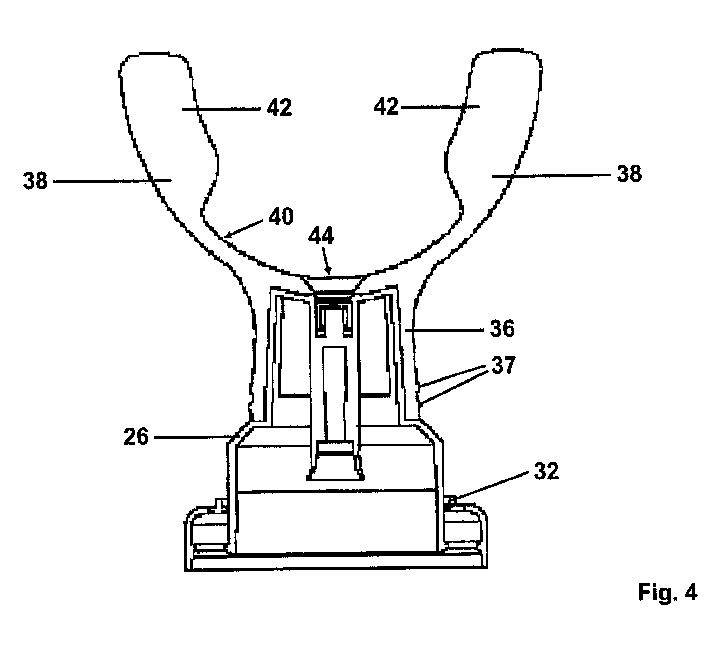

Der Ansatzbereich 36 kann beispielsweise auf dem Sprühkopfkörper 26 verrasten. Auch ist es beispielsweise möglich, das Mundelement 14 über einen anderen Ansatzbereich mit einem Gewinde zu versehen, um es auf den Sprühkopfkörper 26 zu schrauben. Zusätzlich sind in Fig. 4 umlaufende Ringe 37 im Ansatzbereich des Mundelements vorgesehen, die als Griffbereiche für eine manuelle Betätigung vorgesehen sind.

Wie in Fig. 4 zu erkennen, schließen sich an dem Ansatzbereich 36 zwei gekrümmte Flügel 38 an. Die Flügel 38 bilden insgesamt eine konkav gewölbte Anlagefläche 40. An den freien Enden der Flügel 38 sind Bißplatten 42 vorgesehen.

In der Anlagefläche 40 ist eine Austrittsöffnung 44 vorgesehen, über die Flüssigkeit austritt. Der Sprühkopfkörper 26 kann mit einem Abgabevorsprung versehen sein. In dem dargestellten Ausführungsbeispiel fehlt ein Abgabevorsprung, der über die Anlagefläche 40 vorsteht. Bei der Ausgestaltung, bei der ein Abgabevorsprung aus der Anlagefläche 40 vorsteht, ist darauf zu achten, daß der Durchmesser des Abgabevorsprungs kleiner als die Dicke der Bißplatten 42 ist, um eine Aufnahme nicht zu erschweren.

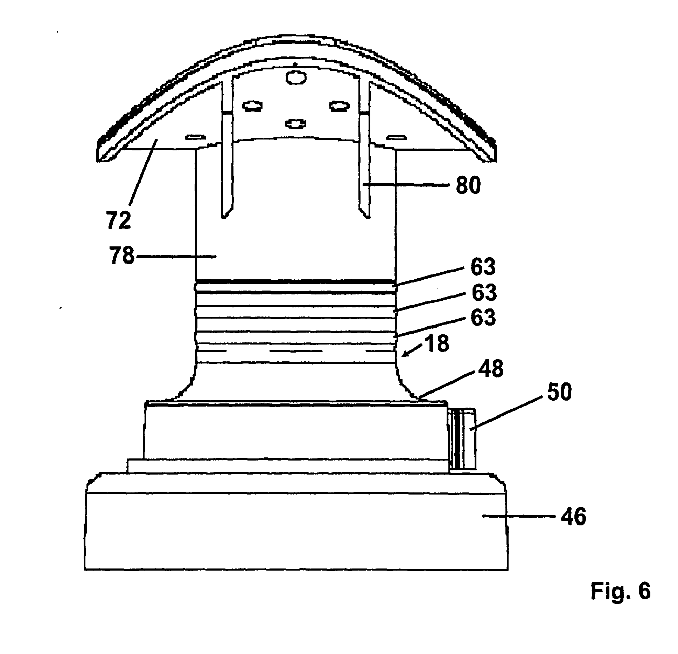

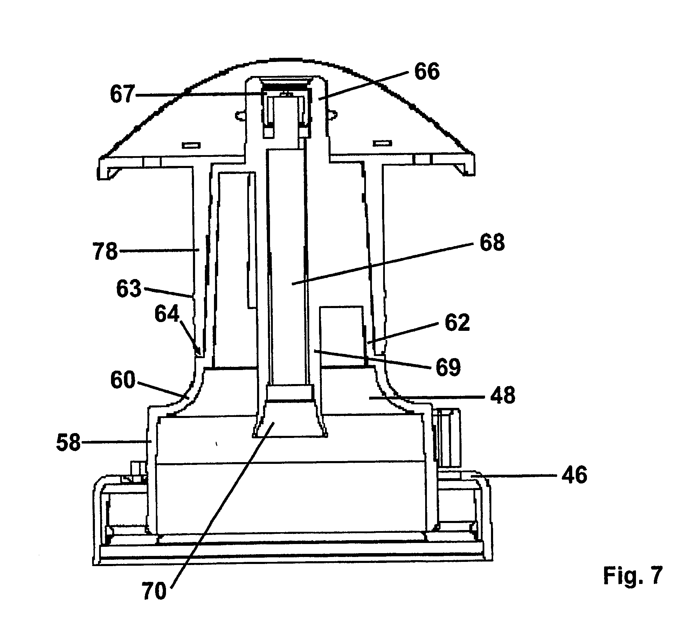

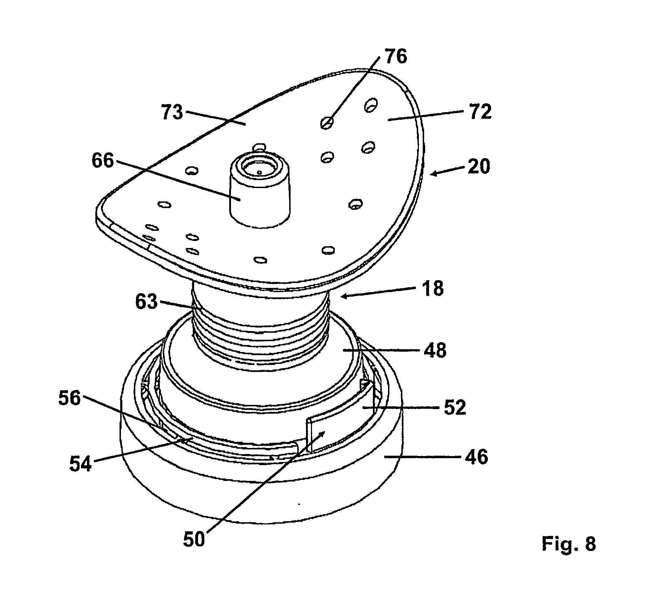

Fign. 6 bis 8 zeigen eine Ausgestaltung des Sprühkopfs 18 mit einem Mundelement 20 gemäß Fig. 2. Der Sprühkopf 18 besitzt einen Sprühkopfkörper 48 und an seinem zum Behälter weisenden Ende einen Befestigungsring 46, der mit dem Behälter verbunden ist. Zwischen Befestigungsring 46 und Sprühkopfkörper 48 ist zusätzlich ein Originalitätsverschluß 50 vorgesehen. Wie in Fig. 8 ersichtlich, besitzt der Originalitätsverschluß 50 eine Lasche 52 und ein umlaufendes Band 54, das über Stege 56 gehalten ist. Die Stege 56 bilden dabei die Verbindung zwischen dem Befestigungsring 46 und dem Sprühkopfkörper 48. Der Sprühkopfkörper besitzt einen glockenförmigen Aufbau mit einem ersten zylindrischen Abschnitt 58, der in einen gebogenen Bereich 60 übergeht. An den gebogenen Bereich 60 schließt sich ein Befestigungsabschnitt 62 an. Der Befestigungsabschnitt 62 besitzt im Übergangsbereich zu dem gebogenen Abschnitt 60 einen Absatz 64. An dem von der Flasche fortweisenden Ende besitzt der Sprühkopfkörper 48 einen Abgabevorsprung 66. Der Abgabevorsprung 66 ist zentral auf dem Sprühkopfkörper 48 geformt und besitzt eine zylindrische Form. In den Abgabevorsprung 66 eingesetzt ist ein Sprühelement 67, das die unter Druck stehende Flüssigkeit beim Austritt versprüht und/oder vernebelt. Der Abgabevorsprung 66 ist über einen in axialer Richtung verlaufenden Austrittskanal mit einem Ventilanschluß 70 verbunden. In dem in Fig. 7 dargestellten Ausführungsbeispiel ist der Sprühkopfkörper 48 mit seinem Austrittskanals 68 für den Anschluß an ein männliches Ventil ausgebildet, wobei der Anschluß über einen sich im Durchmesser erweiternden Anschluß 70 erfolgt.

Das Mundelement 20 besitzt einen Schild 72, der mit Löchern 76 versehen ist. Die Löcher 76 ermöglichen ein Weiteratmen während der Aufnahme von Flüssigkeit. Aus dem Schild 72 steht zentral der Abgabevorsprung 66 vor. Der Schild 72 besitzt einen im wesentlichen ovalen Umriß und ist von dem Sprühbehälter fortgewölbt. Die Form der von dem Schild 72 gebildeten Anlagefläche 73 ist ungefähr an die Form des Mundes eines Benutzers angepaßt.

Der Schild 72 mündet in einem Ansatzbereich 78, der eine zylindrische Form besitzt. Der Ansatzbereich 78 ist, wie aus dem Querschnitt in Fig. 7 erkenntlich, auf den Befestigungsabschnitt 62 aufgesetzt und stößt mit seinem zur Behälterflasche weisenden Ende auf den Absatz 64.

Seitlich abgestützt wird der Schild 72 durch flache Abstützplatten 80, die an der Außenseite des Ansatzbereichs 78 angebunden sind und an der Unterseite des Schilds 72 münden. Weitere Gestaltungsvariationen des Mundelements abweichend von der dargestellten Schildform sind möglich. Diese lassen sich besonders einfach in das Applikatorsystem integrieren, falls Sprühkopf und Mundelement zweiteilig ausgebildet sind.

Bei der Verwendung setzt ein Benutzer den Mund an das Mundelement gemäß Fig. 2 an und umschließt den Abgabevorsprung 22 mit den Lippen. Durch einen geringen Druck mit den Lippen oder den Zähnen wird die Kraft über den Austrittskanal 68 bzw. dessen Mantelwand 69 auf den Anschluß 70 für das Ventil (nicht dargestellt) weitergeleitet und so das Ventil geöffnet. Das austretende Medium tritt über den Austrittskanal 68 in das Sprühelement 67 und gelangt von dort direkt in den Mund des Benutzers. Eine Betätigung von Hand wird durch die umlaufenden Ringe 63 erleichtert.

Aufgrund der besonders einfachen Einnahme der Flüssigkeit eignet sich der erfindungsgemäße Aerosolbehälter insbesondere als orales Applikationssystem für die orale Zuführung von Wirklösungen mit Nähr-/Vitalstoffen und/oder medizinischen Wirkstoffen.

Als Basis für die Wirklösungen eignen sich, einzeln oder in Kombination, beispielsweise die folgenden Inhaltsstoffe:

Wasser, Milch, Alkohol, Fruchtsäfte, Fruchtnektar, und/oder Gemische hiervon.

Als zusätzliche Bestandteile zu dieser Wirklösung können Nähr- und Vitalstoffe sowie medizinische Wirkstoffen und Gemische von diesen vorgesehen sein. So können beispielsweise Kohlenhydrate, einzeln oder in Kombination, z.B. in Form von Einfachzucker (z.B. Monosaccharide wie Glucose, Dextrose, Mannose, Fructose, Ribose, Desoxyribose, Galactose), Zweifachzucker (z.B. Disaccharide wie Saccharose, Lactose, Lactulose, Maltose, Trehalose, Isomaltulose), Dreifachzucker (Trisaccharide wie Melezitose, Raffinose, Umbelliferose) oder Vielfachzucker (Polysaccharide wie Stärke, Zellulose, Glykogen, Chitin, Callose, Fruktane, Dextrane) und Zuckeralkohole (Alditole wie beispielsweise Mannit, Isomalt, Lactit, Sorbit und Xylit, Threit, Erythrit/Erythritol und Arabit) sowie weitere Substanzen, einzeln oder in Kombination, zugesetzt werden. Kohlenhydrate haben unter Einsatz der vorliegenden Erfmdung beispielsweise eine hohe Bedeutung aufgrund ihrer Eigenschaft als Energielieferant, denn sie können über die Zufuhr mit dem erfmdungsgemäßen Applikatorsystem besonders schnell ihre Energie bereitstellen. Weiterhin kann die Bereitstellung von Kohlenhydraten mit dem erfindungsgemäßen Applikatorsystem besonders gut gesteuert werden.

Auch Proteine, einzeln oder in Kombination, wie beispielsweise Molken-Protein, Sojaprotein, Albumin, Muskelprotein, Getreideprotein, Seetang-Protein sowie weitere bekannte Stoffe und Gemische können der Flüssigkeit ebenfalls zugesetzt und mit dem erfindungsgemäßen Applikatorsystem besonders einfach eingenommen werden.

Auch bioaktive Stoffe lassen sich unter Einsatz des Applikatorsystems beispielsweise durch direktes Versprühen oder Vernebeln in den Mund besonders effektiv aufnehmen. Relevante bioaktive Stoffe sind, einzeln oder in Kombination, beispielsweise Inositol, Creatin, Carnitin, Acetylcarnitin, α-Liponsäure, Gamma-Aminobutansäure, beta-alanyl-L-histidine (Carnosin C), Cholin, Cytidine-Diphosphocholine, Dimethyl-Amino-Ethanol, S-Adenosyl-L-Methionin, Quercetin (Flavonoid), Lycopin, Isoflavone, Phytosterole, Glycosaminoglycan, Phosphatidylserin, Lutein sowie weitere bekannte Stoffe und Gemische dieser Zuordnung.

Vitamin- und Vitaminkomponenten, einzeln oder in Kombination, können ebenfalls als Bestandteil der Wirklösung zugesetzt werden, beispielsweise Vitamin A, Vitamin C, Vitamin D, Vitamin E, Vitamin K, Thiamin, Riboflavin, Niacin, Vitamin B6, Folsäure, Vitamin B12, Biotin, Pantothensäure, Coenzym Q10 sowie weitere bekannte Stoffe und Gemische dieser Zuordnung. Vitamine und Vitaminkomponenten können über das erfindungsgemäße orale Applikatorsystem sehr effektiv oral appliziert werden, um lebenswichtige biokatalytische Funktionen, wie die Stoffwechselprozesse des Körpers, ad-hoc oder kontinuierlich zu unterstützen und beispielsweise einer Vitaminunterversorgung vorzubeugen oder Vitaminmangelerscheinigungen zu behandeln.

Auch Fette und ungesättigte Fettsäuren, beispielsweise Omega-3 und Omega-6 sowie weitere bekannte Stoffe und Gemische dieser Zuordnung können einen relevanten Zusatz zur Wirklösung bilden. Fette und Fettsäuren können unter vorliegender Erfindung zum einen effektiv als Energieträger eingesetzt werden, zum anderen können gerade ungesättigte Fettsäuren auch gezielt zur Förderung der Gesundheit appliziert werden. Sie können beispielsweise günstig auf das Herz-Kreislauf-System wirken und das Risiko für Herz-Kreislauf-Erkrankungen positiv beeinflussen.

Stimulanzien und sonstige stimulierende Stoffe, wie beispielsweise Xanthine, Koffein (synthetisch oder natürlich), Theophyllin und Theobromin, Glucuronolakton sowie weitere bekannte Stoffe und Gemische dieser Zuordnung können ebenfalls der Wirklösung zugefügt werden. Sie können erfindungsgemäß sehr effektiv oral appliziert werden, um besonders schnell ihre anregende Wirkung zu entfalten oder auch in Phasen erhöhter körperlicher und geistiger Leistung die Aufmerksamkeit und den Wachheitsgrad sowie die Reaktions- und Konzentrationsfähigkeit positiv zu beeinflussen.

Ferner können viele weitere Substanzen und Gemische, wie beispielsweise die folgenden, einzeln oder in Kombination, Bestandteile der Wirklösung sein und über das erfindungsgemäße System vorteilhaft oral appliziert werden:
- Amino- bzw. Aminosulfonsäuren, einzeln oder in Kombination, wie beispielsweise Tryptophan, Phenylalanin, Tyrosin, Alanin, Arginin, Asparagin, Asparaginsäure, Cystein, Glutamin, Glutaminsäure, Glycin, Histidin, Isoleucin, Leucin, Lysin, Methionin, Prolin, Pyorglutamat, Serin, Threonin, Valin, Taurin sowie weitere bekannte Stoffe und Gemische dieser Zuordnung. Aminosäuren sind wichtige Bausteine von Eiweißen und damit von Körperstrukturen und haben auch spezielle Aufgaben im Stoffwechsel, wie beispielsweise Immunabwehr, Entgiftung oder Hormonsynthese.
- Mineralstoffe, Mineralstoffkomponenten und Spurenelemente, einzeln oder in Kombination, wie beispielsweise Calcium, Kupfer, Eisen, Phosphor, Jod, Magnesium, Zink, Selen, Mangan, Chrom, Molybdän, Chlorid, Kalium, Bor, Nickel, Silizium, Fluor Vanadium, Natrium sowie weitere bekannte Stoffe und Gemische dieser Zuordnung.
- Kräuter und pflanzliche Extrakte und -Zusätze (verarbeitet oder unverarbeitet), einzeln oder in Kombination, wie beispielsweise Ginseng, Tee (zum Beispiel weisser Tee, grüner Tee oder schwarzer Tee), Guarana, Gingko, Echinacea, Zimt, Kamille, Kolanuss, Yerby Mate, Kawa, Yohimbe, Holunderbeere, Traubenkerne, Kurkuma, Mariendistel, Schisandra, Panax Quinquefolium, Reishi, Damiana, Kakao, Carob, Citrus Aurantium, Garcinia Gummi-Gutta, Rotklee, Soja, Acai, Goji, Acerola, Aloevera, Minze, Apfelminze, Aronia, Cranberry, Granatapfel, Carthamuse, Eukalyptus, Honeybush, Hagebutte, Kamillenblüten, Lindenblüten, Löwenzahl, Malve, Melisse, Pfefferminz, Rotbusch, Sanddorn, Shiitake, Zitrone, Zitronen-/Limonengras, sowie weitere bekannte Stoffe und Gemische dieser Zuordnung können ebenfalls zugesetzt werden. Vergorene Pflanzenstoffe, wie beispielsweise Apfelessig, Honig, Kombucha sowie weitere bekannte Stoffe und Gemische dieser Zuordnung.
- Künstliche Süßstoffe, einzeln oder in Kombination, wie Acesulfam, Aspartam, Aspartam-Acesulfam-Salz, Cyclamat, Saccharin, Sucralose, Thaumatin, Neohesperidin, pflanzliche Süßstoffe wie Alitam, Brazzein, Lugdunam, Monellin, Steviosid sowie weitere bekannte Stoffe und Gemische dieser Zuordnung, die den Geschmack positiv beeinflussen können und die Zufuhr der genannten und folgenden Stoffe und Gemische bekömmlicher und wohlschmeckender machen.
- Als Zusatz eignen sich ebenfalls inaktive Substanzen, wie organische und nichtorganische Additive, einzeln oder in Kombination, die für die Herstellung der Wirklösungen zur Verabreichung mit dem erfindungsgemäßen oralen Applikatorsystem nützlich sind, wie beispielsweise konventionelle Füllstoffe, Verdickungsmittel, Extender oder Bindemittel wie Laktose, Mannitol, Sorbitol, Zellulose, Kalciumphospat, Stärke, Gelatine, Tragant, Methylzellulose, Hydroxypropylmethylzellulose, Sodiumcarboxymethylzellulose, Polyvinylpyrrolidone, und Substanzen zur Beeinflussung der Flies- und Gleitfähigkeit, wie beispielsweise Silica, Talk, Kalciumstearate, Magensiumstearate, Polyethylenglyko. Stabilisatoren sowie disintegrative Substanzen wie die genannte Stärke oder auch beispielsweise Carboxymethylstärke, Polyvinylpyrrolidone, Agar, Alginsäure oder auch Salze, wie Sodium-Alginat, können auch zugesetzt werden. Als Konservierungsmittel eignet sich beispielsweise Formic Acid (Ameisensäure) und weitere bekannte lebensmittelkonforme Stoffe und Gemische dieser Zuordnung.
- Ätherische Öle, einzeln oder in Kombination, wie Anisöl, Basilikumöl, Bayöl, Birkenöl, Cajeputöl, Campher, Dillöl, Eukalyptusöl, Geranienöl, Honigöl, Hopfenöl, Karottensamenöl, Kümmelöl, Lavendelöl, Mandarinenöl, Melissenöl, Orangenöl, Patschuliöl, Pfefferminzöl, Pimentöl, Rosenöl, Sandelholzöl, Teebaumöl, Thymianöl, Zimtöl, Öl aus Zitrusfrüchten sowie weitere bekannte Stoffe und Gemische dieser Zuordnung können darüber hinaus zugesetzt werden. Ihre Bedeutung unter vorliegender Erfindung liegt beispielsweise darin, daß sie flüchtig und leicht verdampfend sind und somit effektiv zugeführt werden können. So gibt es ätherische Öle, wie Eukalyptus oder Menthol, die eine hervorragende schleimlösende Wirkung bei Katarrhen der oberen Atemwege haben und effektiv mit der vorliegenden Erfindung oral appliziert werden können. Ätherische Öle können bei der vorliegenden Erfindung auch hervorragend als anregende Duftstoffe zur Ergänzung der Wirklösung eingesetzt werden.

- So eigenen sich auch Duft- und Geschmacksstoffe insgesamt als Bestandteil der Wirklösung, einzeln oder in Kombination, beispielsweise sogenannte FTNF Aromen, WONF Aromen, natürliche Aromen, naturidentische Aromen, künstliche Aromen, Aromaextrakte sowie weitere bekannte Stoffe und Gemische dieser Zuordnung, die Geschmack und Geruch positiv beeinflussen können und die Zuführ der genannten und folgenden Stoffe und Gemische als auch die Wirklösung insgesamt bekömmlicher und wohlschmeckender machen.
- Antioxidantien und Säuerungsmittel wie Ascorbinsäure, Natrium Ascorbate, Natrium Erythorbate, Isoascorbinsäure, Apfelsäure, Zitronensäure, Natriumcitrate, Phosphorsäure, Weinsäure, Weinstein, Glucone-delta-lactone sowie weitere bekannte Stoffe und Gemische dieser Zuordnung können zur Verbesserung der Lagerzeit und/oder der Haltbarkeit zugesetzt werden.
- Konservierungsstoffe wie Benzoesäure, Kaliumbenzoat, Natriumbenzoate, Nisin sowie weitere bekannte Stoffe und Gemische dieser Zuordnung können unter Einsatz vorliegender Erfindung beispielsweise die Haltbarkeit weiterer Stoffe und Gemische positiv beeinflussen.
- Farbstoffe wie Tartrazin (E 102), Chinolingelb (E104), Gelborange S (E 110), Karmin bzw. Cochenille (E 120), Allurarot AC (E129), Patentblau VF (E 131), Indigotin (E 132), Brillantblau FCF (E133), Kupfer-Chlorophylle (E141), Grün S (E142), Ammonsulfit-Zuckerkulör (E150d), Brillantschwarz BN (E151), Carotine (E160a), Azorubin (E122) sowie weitere bekannte Stoffe und Gemische dieser Zuordnung können in der Flüssigkeiten gelöst werden, um einen ansprechenden Gesamteindruck herzustellen.
- Medizinische Substanzen, Wirkstoffe und Wirkstoffkombinationen können als funktionale Bestandteile der Wirklösung hinzugefügt werden, um mit dem erfindungsgemäßen Applikatorsystem oral zugeführt zu werden. So können unter anderem Wirkstoffe wie Acetylsalicylsäure (beispielsweise schmerzlindernde, entzündungshemmende, antirheumatische, gerinnungshemmende oder auch fiebersenkende Wirkung), Paracetamol (beispielsweise schmerzlindernd und fiebersekend), Antitussiva, Sedativa, appetithemmende Anorektika, potenzfördernde natürliche und chemische Wirkstoffe sowie weitere bekannte Substanzen und Wirkstoffkombinationen medizinischer Zuordnung eingesetzt werden.

Die vorstehende Wirklösung und ihre Zusammensetzung ist lediglich beispielhaft.

## Patentansprüche

1. Applikatorsystem mit einem unter Druck stehenden Medium und einem Ventil zum Austrag des Mediums,
wobei der Sprühkopf (12, 18) einen Sprühkopfkörper (26, 48) aufweist, der einen mit dem Ventil verbundenen Austrittskanal (68) besitzt und eine Austrittsöffnung, in der der Austrittskanal mündet, und **dadurch gekennzeichnet ist,**
**dass** ein Mundelement (14, 20) vorgesehen ist, das auf dem Sprühkopfkörper (26, 48) sitzt, wobei die Austrittsöffnung und das Mundelement (14, 20) relativ zueinander angeordnet sind, um die Austrittsöffnung an den Mund eines Benutzers führen und über das Mundelement das Ventil betätigen zu können.

2. Applikatorsystem nach Anspruch 1, **dadurch gekennzeichnet, daß** der Sprühkopfkörper einen Abgabevorsprung aufweist.

3. Applikatorsystem nach Anspruch 2, **dadurch gekennzeichnet, daß** das Mundelement (14, 20) eine zentrale Öffnung aufweist, durch die der Abgabevorsprung (22, 66) vorsteht.

4. Applikatorsystem nach einem der Ansprüche 1 bis 3, **dadurch gekennzeichnet, daß** an dem Sprühkopf und/oder dem Mundelement mindestens ein Griffbereich (37, 63) für eine manuelle Betätigung vorgesehen ist.

5. Applikatorsystem nach einem der Ansprüche 1 bis 4, **dadurch gekennzeichnet, daß** das Mundelement einen gewölbten Schild (72) aufweist.

6. Applikatorsystem nach Anspruch 5, **dadurch gekennzeichnet, daß** der Schild flache von der Austrittsöffnung fortweisende Flügel (38) aufweist.

7. Applikatorsystem nach Anspruch 6, **dadurch gekennzeichnet, daß** die Flügel (38) eine Beißleiste (42) aufweisen.

8. Applikatorsystem nach Anspruch 5, **dadurch gekennzeichnet, daß** der Schild (72) als Anlagefläche (73) für die Lippen eines Benutzers ausgebildet sind.

9. Applikatorsystem nach einem der vorangehenden Ansprüche, **dadurch gekennzeichnet, daß** das Mundelement eine in axialer Richtung des Aerosolbehälters auf das Mundelement aufgebrachte Kraft auf das Ventil weiterleitet.

10. Applikatorsystem nach einem der vorangehenden Ansprüche, **dadurch gekennzeichnet, daß** der Sprühkopfkörper eine umlaufende Wand besitzt, die ein Befestigungsabschnitt (62) für das Mundelement bildet.

11. Applikatorsystem nach dem vorangehenden Anspruch, **dadurch gekennzeichnet, daß** das Mundelement einen zylindrischen Ansatzbereich besitzt, der eine umlaufende Wand aufweist, deren Innenseite an dem Befestigungsabschnitt (62) für das Mundelement anliegt.

12. Applikatorsystem nach Anspruch 10, **dadurch gekennzeichnet, daß** der Befestigungsabschnitt einen umlaufenden Absatz (64) aufweist, an dem der Ansatzbereich anliegt.

13. Applikatorsystem nach einem der vorangehenden Ansprüche, **dadurch gekennzeichnet, daß** ein Sprühelement (67) in dem Austrittskanal angeordnet ist.

14. Applikatorsystem nach Anspruch 13, **dadurch gekennzeichnet, daß** das Sprühelement (67) das austretende Medium zerstäubt.

15. Applikatorsystem nach Anspruch 13 oder 14, **dadurch gekennzeichnet, daß** das Sprühelement (67) als Einsatzkörper ausgebildet ist, der im Abgabevorsprung angeordnet ist.

## Claims

1. An applicator system with a pressurized medium and a valve for discharging the medium,
wherein the spray head (12, 18) has a spray head body (26, 48), which has a discharge channel (68) connected to the valve, and a discharge opening into which the discharge channel runs out, and is **characterised in that**
a mouth element (14, 20) is provided, which rests on the spray head body (26, 48), wherein the discharge opening and the mouth element (14, 20) are disposed with respect to each other such as to guide the discharge opening to the mouth of an user,
and to be able to actuate the valve via the mouth element.

2. The applicator system according to claim 1, **characterised in that** the spray head body has a delivery overhang.

3. The applicator system according to claim 2, **characterised in that** the mouth element (14, 20) has a central opening, through which the delivery overhang (22, 66) projects.

4. An applicator system according to any one of claims 1 to 3, **characterised in that** at least one grip area (37, 63) for manual operation is provided on the spray head and/or the mouth element.

5. An applicator system according to any one of claims 1 to 4, **characterised in that** the mouth element has a vaulted shield (72).

6. The applicator system according to claim 5, **characterised in that** the shield has flat wings (38) which point away from the discharge opening.

7. The applicator system according to claim 6, **characterised in that** the wings (38) have a bite strip (42).

8. The applicator system according to claim 5, **characterised in that** the shield (72) is configured as a bearing surface (73) for the lips of a user.

9. An applicator system according to any one of the preceding claims, **characterised in that** the mouth element forwards a force to the valve which is applied to the mouth element in the axial direction of the aerosol container.

10. An applicator system according to any one of the preceding claims, **characterised in that** the spray head body has a circulating wall which forms a fastening portion (62) for the mouth element.

11. An applicator system according to any one of the preceding claims, **characterised in that** the mouth element has a cylindrical joining area which has a circulating wall whose inner side bears against the fastening portion (62) for the mouth element.

12. The applicator system according to claim 10, **characterised in that** the fastening portion has a circulating shoulder (64), against which bears the joining area.

13. An applicator system according to any one of the preceding claims, **characterised in that** a spray element (67) is disposed in the discharge channel.

14. The applicator system according to claim 13, **characterised in that** the spray element (67) atomizes the leaving medium.

15. An applicator system according to claim 13 or 14, **characterised in that** the spray element (67) is configured as an insert body, which is disposed in the delivery overhang.

## Revendications

1. Système applicateur avec un médium sous pression et une soupape pour décharger le médium,
dans lequel la tête de pulvérisation (12, 18) a un corps de tête de pulvérisation (26, 48), qui a un conduit de décharge (68) raccordé à la soupape et une ouverture de décharge dans laquelle le conduit de décharge débouche, et qui est **caractérisé en ce que**
un élément de bouche (14, 20) est pourvu, qui repose sur le corps de tête de pulvérisation (26, 48), l'ouverture de décharge et l'élément de bouche (14, 20) étant disposé l'un à l'autre de façon à pouvoir guider l'ouverture de décharge à la bouche d'un utilisateur et actionner la soupape au travers de l'élément de bouche.

2. Système applicateur selon la revendication 1, **caractérisé en ce que** le corps de tête de pulvérisation a une saillie de délivrance.

3. Système applicateur selon la revendication 2, **caractérisé en ce que** l'élément de bouche (14, 20) a une ouverture centrale à travers la saillie de délivrance (22, 66) fait saillie.

4. Système applicateur selon l'une quelconque des revendications 1 to 3, **caractérisé en ce que** au moins une région de manette (37, 63) pour opération à main est pourvue sur la tête de pulvérisation et/ou l'élément de bouche.

5. Système applicateur selon l'une quelconque des revendications 1 to 4, **caractérisé en ce que** l'élément de bouche a un bouclier (72) en arc.

6. Système applicateur selon la revendication 5, **caractérisé en ce que** le bouclier a des ailettes (38) plats qui montrent en dehors de l'ouverture de décharge.

7. Système applicateur selon la revendication 6, **caractérisé en ce que** les ailettes (38) ont une bordure de dentition (42).

8. Système applicateur selon la revendication 5, **caractérisé en ce que** le bouclier (72) est formé comme une surface d'appuy (73) pour les lèvres d'un utilisateur.

9. Système applicateur selon l'une quelconque des revendications précédentes, **caractérisé en ce que** l'élément de bouche transmet une force à la soupape qui est appliquée à l'élément de bouche dans la direction axiale du réservoir d'aérosol.

10. Système applicateur selon l'une quelconque des revendications précédentes, **caractérisé en ce que** le corps de tête de pulvérisation a une paroi circonférentielle qui forme une partie d'attache (62) pour l'élément de bouche.

11. Système applicateur selon l'une quelconque des revendications précédentes, **caractérisé en ce que** l'élément de bouche a une région de débord cylindrique qui a une paroi circulante sur la circonférence dont le côté est adjacent à la partie d'attache (62) pour l'élément de bouche.

12. Système applicateur selon la revendication 10, **caractérisé en ce que** la partie d'attache a une épaule circonférentielle (64), sur laquelle la région de débord repose.

13. Système applicateur selon l'une quelconque des revendications précédentes, **caractérisé en ce que** un élément de pulvérisation (67) est disposé dans le conduit de décharge.

14. Système applicateur selon la revendication 13, **caractérisé en ce que** l'élément de pulvérisation (67) vaporise le médium émergeant.

15. Système applicateur selon la revendication 13 or 14, **caractérisé en ce que** l'élément de pulvérisation (67) est configuré comme un corps insérable, qui est disposé dans la saillie de délivrance.
